# DEMANDE DE BREVET EUROPEEN

(11) **EP 3 456 277 A1**
(43) Date de publication de la demande: **20.03.2019**
(21) Numéro de dépôt: 18195282.1
(22) Date de dépôt: 18.09.2018
(51) Int. Cl.: A61B 17/86, A61M 39/20, A61M 39/02, A61F 2/50

(54) **DISPOSITIF D'ANCRAGE EPITHELIAL**

(30) Priorité: 18.09.2017 FR 1700944
(71) Demandeur: Zinzindohoue-Marsaudon, Frank, 92600 Asnieres-sur-Seine (FR)
(72) Inventeur: Zinzindohoue-Marsaudon, Frank, 92600 Asnieres-sur-Seine (FR)
(74) Mandataire: Louiset, Raphaël

(57) **Abrégé**

Dispositif d'ancrage épithélial composé d'un manchon creux (**1**) traversé par au moins un conduit (**2**) interne avec au moins une ouverture (**3**, **4**) à chacune de ses extrémités externe (**5** ou **A**) et interne (**6** ou **B**) qui permet l'introduction d'au moins un tube, amovible ou fixe, plein ou creux, et qui est extérieurement fait au moins en partie de composite carbone/carbone, permettant une inclusion et un ancrage épithélial pour protéger un organisme humain ou animal du risque d'infection depuis l'extérieur.

## Description

### Domaine de l'invention

La présente invention concerne un système d'ancrage épithélial avec un risque infectieux très faible pour des dispositifs médicaux. La présente invention est un manchon plein, ou creux traversé par un conduit interne. Elle est recouverte extérieurement sur une surface variable d'un matériau ayant les propriétés et caractéristiques d'épithéliointégration qui permettent au carbone d'être épithéliointégré. Ces propriétés d'épithéliointégration permettent une implantation dans un épithélium sous la forme d'une inclusion, avec un ancrage durable. La présente invention empêche la survenue d'infections à son interface avec l'épithélium. L'invention peut servir de conduit d'introduction intracorporelle pour faire passer des tubes pleins ou creux à travers un conduit interne qui la traverse vers l'intérieur d'un organisme vivant. Le dispositif selon l'invention ancré dans un épithélium au sein duquel il est inclus peut servir à accrocher un dispositif extérieur auquel il est fixé. Le dispositif selon l'invention est posé durablement avec un risque infectieux très faible à l'intérieur ou au travers d'un épithélium pour réaliser des transferts de fluides ou d'énergie entre l'intérieur et l'extérieur d'un organisme avec un risque infectieux très faible, ou pour créer un point d'ancrage à la surface de l'épithélium.

### Etat de la technique

Tous les dispositifs médicaux qui servent de voie d'échange externe-interne doivent traverser un épithélium et sont colonisés par des micro-organismes à partir de leur point d'entrée à la surface de l'épithélium. Le risque infectieux par contamination depuis ce point d'entrée jusque dans l'organisme est important et augmente avec le temps. Tous les systèmes actuels finissent par être colonisés par des micro-organismes puis l'infection se développe et oblige à les retirer.

Pour minimiser le risque infectieux et ses conséquences la plupart des dispositifs médicaux utilisés comme voies d'échange sont temporaires, facilement explantables et se limitent souvent à des échanges de fluides (perfusion), plus rarement à des transferts de signaux électriques. Cela limite le développement de dispositifs médicaux intracorporels de longue durée qui nécessitent une quantité importante d'énergies électrique ou lumineuse, ou de fluides. Cela limite les échanges d'énergies électrique ou lumineuse, ou de fluides de part et d'autre d'un épithélium, entre l'intérieur et l'extérieur du corps.

Seuls les dispositifs médicaux recouverts extérieurement de matériaux ayant la propriété d'ostéointégration peuvent à la fois être exposés durablement au milieu extérieur et être implantés dans un os avec un faible risque infectieux périprothétique. Cela est dû à l'ostéointégration qui est une inclusion et un ancrage de la surface externe d'un dispositif médical implantable directement dans l'os environnant sans interposition fibreuse ni espace périprothétique. Lors de cette inclusion, la jonction entre l'os et l'implant ostéointégré est si intime qu'elle forme durablement une barrière efficace contre le développement de micro-organismes dans cette interface et l'infection est bloquée au contact de l'os même lorsque la partie extérieure traversant la peau est colonisée par des micro-organismes. L'inconvénient des voies de communication ostéointégrées est la nécessité d'un l'os immédiatement sous-épithélial ce qui réduit les zones éligibles.

### Exposé de l'invention

L'ostéointégration est un cas particulier d'intégration tissulaire de matériaux biocompatibles. Par exemple, le titane est ostéointégrable. Nous avons découvert que du carbone, des fibres de carbones, et des composites carbone/carbone sont épithéliointégrables. Nous définissons l'épithéliointégration comme une inclusion et un ancrage durable du carbone dans un épithélium sans interposition fibreuse ni espace périprothétique au niveau de la partie en contact avec l'épithélium traversé depuis l'extérieur. La biocompatibilité du carbone est connue depuis la préhistoire puisqu'on trouve sur des momies des tatouages réalisés avec des particules de graphite obtenu à partir de la combustion de composés organique comme du bois, sans rejet des particules ni infection de la peau. Cette pratique existe toujours et les examens histologiques épithéliaux de tatouages contemporains montrent la même épithéliointégration lorsque des particules de carbone sont utilisées comme pigment. Le charbon de bois est constitué de carbone graphite friable formé d'une structure comparable aux structures réticulées des fibres de carbone et des composites carbone/carbone. Nous avons montré que des fibres de carbone et des composites carbone/carbone traversant les épithéliums sont biocompatibles et épithéliointégrés après implantation chez l'animal. Cette épithéliointégration après cicatrisation est clinique comme le montre l'absence de signes infectieux et de signes inflammatoires au point d'insertion sur la peau ou les muqueuses, et est histologique comme le montre l'absence de réaction à corps étranger et l'absence d'infiltrat infectieux et d'infiltrat inflammatoire au contact de fibre de carbone ou de composite carbone/carbone lors d'un examen au microscope. Cette épithéliointégration est durable.

Une implantation sous contraintes mécaniques latérales positives, vissée dans l'épithélium par exemple, permet une fixation en impaction de la partie recouverte extérieurement sur une surface de longueur variable de carbone, de fibres de carbone ou de composite carbone/carbone épithéliointégrable. C'est un facteur non nécessaire mais qui favorise une bonne épithéliointégration lors de l'implantation et favorise l'inclusion et l'ancrage épithélial. Ainsi il n'y a pas de risque de propager une infection le long de la face externe du dispositif selon l'invention qui sert d'implant épithéliointégré lorsqu'une extrémité affleure ou dépasse de l'épithélium dans le milieu extérieur. Un dispositif selon la présente invention recouvert extérieurement de carbone sur la surface en contact avec l'épithélium peut être fixé dans une structure anatomique sous-jacente dure ou fixe, par exemple de l'os ou des ligaments, pour diminuer sa mobilité. Un dispositif selon la présente invention peut être strictement intra-épithélial et ne pas pénétrer plus profondément dans l'organisme pour servir à la fixation cutanée d'un postiche ou d'une prothèse. Au terme de la cicatrisation la surface externe du dispositif selon l'invention en contact avec l'épithélium est totalement épithéliointégrée et il n'y a plus d'espace laissant passer des micro-organismes à son contact dans l'épithélium et vers l'intérieur de l'organisme et donc le risque infectieux résiduel est quasi nul. Cela permet d'utiliser la présente invention pour faire pénétrer dans l'organisme au moins un tube plein ou creux sans risque infectieux significatif de contamination bactérienne.

Le dispositif selon l'invention est partiellement recouvert extérieurement ou entièrement fait d'un matériau ayant les propriétés et caractéristiques d'épithéliointégration du carbone que nous avons décrites ci-dessus ou est partiellement recouvert extérieurement de carbone ou entièrement fait en carbone, en fibres de carbone ou en composites carbone/carbone. Il a la forme d'un manchon plein, creux, mono ou multi-perforé entre deux extrémités ouvertes ou borgnes. Le dispositif selon l'invention est orientable selon un axe A-B avec une extrémité externe vers la surface de l'épithélium et une extrémité interne intracorporelle. L'extrémité intra-épithéliale est de longueur variable pour servir de système d'ancrage s'arrêtant dans l'épithélium, ou allant plus profondément par exemple jusque dans un os pour diminuer sa mobilité. Le dispositif selon l'invention peut être plus large dans le plan perpendiculaire à l'axe A-B qu'il n'est haut selon l'axe A-B. Le dispositif selon l'invention est une voie de passage qui sert de conduit d'introduction intracorporel transépithélial et en même temps trans-osseux s'il traverse au-dessous de l'épithélium l'une des corticales d'un os pour atteindre le canal médullaire, ou plus profondément les deux corticales d'un os. Le dispositif selon l'invention est destiné à ce qu'un ou plusieurs conduits internes puissent être traversés entre les deux extrémités suivant l'axe A-B par un ou plusieurs tubes amovibles ou fixes, pleins ou creux et de longueur variable ayant avec lui un contact étanche pour empêcher tout passage de micro-organisme vers l'intérieur de l'organisme.

Le dessin annexé illustre le dispositif selon l'invention :

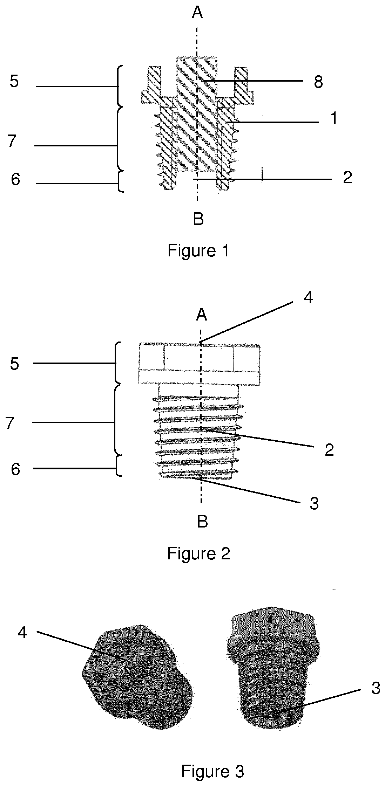

- La **figure 1** représente une vue externe du dispositif de l'invention ;
- La **figure 2** représente une vue en coupe montrant le conduit interne fileté pour recevoir de façon étanche un tube ;
- La **figure 3** donne deux représentations en perspective du dispositif de l'invention.

En référence à ces dessins, le dispositif selon l'invention est un manchon **1,** fileté sur une hauteur variable de sa face externe dans lequel s'ouvre un conduit interne **2** qui le traverse. Le dispositif selon l'invention comporte une extrémité **5** (ou **A**) externe, un segment **7** intermédiaire qui porte le filetage, et une extrémité **6** (ou **B**) interne. Le conduit **2** interne qui traverse le dispositif selon l'invention est fileté sur une longueur variable pour permettre l'impaction occlusive lors de l'obturation par un tube **8** fileté. Le manchon **1** est ouvert à l'extrémité **A** externe par un orifice **3** externe et à l'extrémité **B** externe par un orifice **4** interne.

Selon une variante non illustrée, le manchon **1** peut être recouvert extérieurement sur une hauteur variable du segment **7** par une couche de carbone épithéliointégrable.

Selon une variante non illustrée le manchon **1** peut être ouvert latéralement soit à l'extrémité **5** externe, soit à l'extrémité **6** interne, soit aux deux extrémités **5, 6** pour former un ou plusieurs orifices latéraux d'inclinaison variable et non axiaux.

Selon une variante non illustrée, le manchon **1** peut être de forme externe cylindrique, tronconique, convexe ou concave.

Selon une variante non illustrée le manchon **1** peut être plein et n'être pas traversé par un conduit interne.

Selon une variante non illustrée le manchon **1** peut être de forme extérieure hyperboloïde à une nappe.

Selon une variante non illustrée le dispositif **1** selon l'invention peut être de forme extérieure circulaire, elliptique ou polygonale à faces planes, concaves ou convexes dans le plan perpendiculaire à l'axe A-B.

Selon une variante non illustrée l'extrémité **5** (ou **A**) du manchon **1** peut être usinée de sorte à permettre d'appliquer une force de torsion axiale au moyen d'un tournevis, d'une clé-multi-pans externe de type clé femelle par exemple une clé à pipe ou une clé plate, ou d'une clé-multi-pans externe de type clé mâle par exemple une clé convexe multi-pans type Allen ou Torx (marques déposées).

Selon une variante non illustrée, l'extrémité **5** (ou **A**) externe est munie de bourrelets périphériques servant à faciliter la prise lors de manoeuvre de pose, d'extraction ou de la fixation à des éléments extra-corporels.

Selon une variante non illustrée, l'extrémité **6** interne (du côté **B**), l'extrémité **5** externe (du côté **A**), ou les deux extrémités **5, 6** peuvent être munies d'un système de fixation convexe (mâle), concave (femelle) ou magnétique.

Selon une variante non illustrée l'extrémité **5** (ou **A**) externe est une pièce indépendante du segment **7** intermédiaire et de l'extrémité **6** (ou **B**) interne du manchon **1,** auxquels elle peut être fixée secondairement.

Selon une variante non illustrée le manchon **1** selon l'invention est réduit à la longueur du segment **7** intermédiaire sans les extrémités **5** (ou **A**) et **6** (ou **B**).

Selon une variante non illustrée la surface externe du segment **7** intermédiaire du manchon **1** peut être lisse, rugueuse, dépolie, striée ou couverte d'aspérités sur tout ou partie de sa longueur.

Selon une variante non illustrée la surface externe du segment **7** intermédiaire du manchon **1** peut n'être pas filetée.

Selon une variante non illustrée l'extrémité **6** (ou **B**) interne du manchon **1** peut ne pas avoir d'ouverture **3** interne et le manchon **1** contient alors une cavité.

Selon une variante non illustrée, le manchon **1** peut être traversé par plusieurs conduits **2** internes.

Selon une variante non illustrée le conduit **2** interne du manchon **1** ne comporte pas de filetage mais un ou plusieurs bourrelets qui permettent de clipser ou d'emboutir un tube **8** plein ou creux de longueur variable. La forme extérieure du tube **8** peut être congruente avec la surface du conduit interne pour une occlusion étanche.

Selon une variante non illustrée l'extrémité externe **5** du manchon **1** comporte extérieurement un filetage qui permet de le visser, ou comporte extérieurement un ou plusieurs bourrelets qui permettent de le clipser ou l'emboutir.

Selon une variante non illustrée les surface des extrémités 5, 6 orientée respectivement vers **A** et **B** peuvent ne pas être parallèles entres elles, ni perpendiculaires à l'axe A-B.

Selon une variante non illustrée la longueur selon suivant l'axe **A-B** est variable qu'elle que soit sa largeur dans le plan perpendiculaire à l'axe **A-B.**

Domaines d'application pour introduire de tubes dans un organisme.

De nombreux dispositifs médicaux sont alimentés par de l'énergie électrique en neurologie et en cardiologie par exemple. Une motorisation des articulations pour compenser des déficits neuromusculaires pourrait permettre une alimentation électrique de forte puissance grâce à ce dispositif. Dans ce cas un ancrage intra osseux peut permettre de passer les tubes d'alimentation en énergie jusque dans le canal médullaire.

Du fait du risque infectieux ces dispositifs sont habituellement autonomes grâce à des batteries et ne sont pas physiquement reliés à l'extérieur de l'organisme par un câblage ou des conduits. Les batteries ne peuvent être rechargées durant la durée de l'implantation intracorporelle et nécessitent une opération chirurgicale pour leur changement. L'encombrement des batteries implantables impose une limite de poids et de volume. Cela limite la durée d'autonomie des dispositifs médicaux électriques, et leur puissance maximale disponible. C'est pourquoi ces dispositifs délivrent majoritairement des impulsions électriques et sont rarement motorisés ce qui limite leur champ d'applications.

Une motorisation des articulations en cas de déficit neuro-musculaire n'est pas possible actuellement du fait de la nécessité d'une alimentation énergétique durable, de forte puissance et éventuellement continue. Une motorisation articulaire pourrait être développée grâce à ce dispositif pour équiper des patients atteints de handicap moteur. Dans ce cas un ancrage intra osseux pourrait permettre de passer les tubes d'alimentation en énergie par le canal médullaire.

Le dispositif selon l'invention pourrait servir à créer différent type de connectiques comme des prises de courant pour charger des batteries sans limite de charge électrique ou à programmer un dispositif électronique sur des connexions filaires et donc sécurisées car non accessible à distance par des tiers non autorisés. Ces connectiques pourraient être au standard USB 1, 2 ou 3.

Le dispositif selon l'invention permet de protéger du risque infectieux un système filaire transépithélial en le faisant passer par le conduit **2** interne grâce à l'épithéliointégration de la surface extérieure du manchon **1.** Il permet alors une conduction électrique sans risque infectieux entre les milieux extra et intra corporel y compris par de nombreux faisceaux sans limite de puissance ni de durée. Le dispositif selon l'invention est particulièrement adapté pour l'alimentation électrique des dispositifs médicaux intracorporels, y compris des moteurs électriques, pour la conduction de courants et impulsions électriques faisant partie d'un circuit électronique, ou encore la charge discontinue de batteries intracorporelles connectées de façon séquentielle à une source électrique externe.

De plus en plus de systèmes pour une assistance cardio-vasculaire, endocrinienne, nutritionnelle, ou d'épuration rénale nécessitent des échanges de fluides entre l'extérieur et l'intérieur du corps. Tous ses systèmes nécessitent de traverser un épithélium avec des tubes de calibres différents. La traversée épithéliale de ces tubes est le siège d'infections qui peuvent se propager à l'intérieur du corps le long des tubes. Le dispositif selon l'invention permet en les faisant passer par le conduit **2** interne de les protéger du risque infectieux grâce à l'épithéliointégration de la face extérieure du manchon **1.**

On fait ainsi passer par le manchon épithéliointégrable, sans risque infectieux, des tubes comme des fils conducteurs électriques des dispositifs médicaux implantables ou des tuyaux pour des fluides échangés avec un dispositif médical ou directement avec l'organisme humain ou animal. Cela permettrait des échanges sans limite de volume, de débit, de charge électrique ni de durée avec l'intérieur de l'organisme sans risque infectieux.

Domaines d'application pour l'ancrage épithélial de dispositifs extra-corporels ou à la fois extra et intracorporels.

Le port de postiches capillaires ou de prothèse en cas d'amputation (oreille, nez, membre, etc.) pose le problème de leur fixation cutanée qui fait appel à des systèmes élastiques ou des encollages inconfortables ou peu fiables. Le système selon l'invention pourrait constituer d'encrage intra-épithélial de boutons pressions sur lesquels fixer les postiches et prothèses, ou l'encrage monobloc des postiches ou prothèses, éventuellement en combinant un ancrage osseux sous-jacent pour supporter des contraintes de poids.

## Revendications

1. Dispositif d'ancrage épithélial **caractérisé en ce qu'**il se compose d'un manchon creux (**1**) traversé par au moins un conduit (**2**) interne avec au moins une ouverture (**3**, **4**) à chacune de ses extrémités externe (**5** ou **A**) et interne (**6** ou **B**) qui permet l'introduction d'au moins un tube, amovible ou fixe, plein ou creux, et qui est extérieurement fait au moins en partie de composite carbone/carbone, permettant une inclusion et un ancrage épithélial pour protéger un organisme humain ou animal du risque d'infection depuis l'extérieur.

2. Dispositif selon la revendication 1, **caractérisé en ce qu'**il est traversé par au moins un conduit (**2**) interne muni de filetage ou de bourrelet(s) permettant la fixation congruente d'au moins un tube.

3. Dispositif selon la revendication 1, **caractérisé en ce qu'**il a une surface externe lisse, filetée, rugueuse, dépolie, striée, ou couverte d'aspérités sur tout ou partie de sa longueur dont la forme générale est cylindrique, tronconique, hyperboloïde à une nappe, concave ou convexe.

4. Dispositif selon la revendication 1, **caractérisé en ce que** la forme extérieure dans le plan perpendiculaire à l'axe (**A-B**) du manchon (1) est circulaire, elliptique ou polygonale à faces planes, concaves ou convexes.

5. Dispositif selon la revendication 1, **caractérisé en ce qu'**il est muni à son extrémité (**5** ou **A**) et/ou à son extrémité (**6** ou **B**) interne, d'un système de fixation convexe (mâle), concave (femelle) ou magnétique.

6. Dispositif selon la revendication 1, **caractérisé en ce qu'**il est monobloc ou en plusieurs parties.
